# EUROPEAN PATENT SPECIFICATION

(11) **EP 1 170 372 B1**
(45) Date of publication and mention of the grant of the patent: **19.04.2006**
(21) Application number: 00114560.6
(22) Date of filing: 06.07.2000
(51) Int. Cl.: C12N 15/63, C12N 15/12, C07K 14/47

(54) **Regulatory sequences of the human MCP-1 gene**
Regulatorische Sequenzen des humanen MCP-1 Gens
Séquences régulatrices du gène MCP-1

(43) Date of publication of application: 09.01.2002
(73) Proprietor: Deutsches Krebsforschungszentrum Stiftung des Öffentlichen Rechts, 69120 Heidelberg (DE)
(72) Inventor: Rösl, Dr. Frank, 67141 Neuhofen (DE); Soto, Dr. Ubaldo, 69121 Heidelberg (DE); Coy, Dr. Johannes, 63762 Grossostheim (DE); Finzer, Dr. Patrick, 68165 Mannheim (DE); Delius, Dr. Hajo, 69221 Dossenheim (DE); Poustka, Prof. Annemarie, 69120 Heidelberg (DE); zur Hausen, Prof. Dr. Harald, 69583 Waldmichelbach (DE); Patzelt, Andrea, 69214 Eppelheim (DE)
(74) Representative: Schüssler, Andrea

(56) References cited:
- EMBL Database, Heidelberg, FRG Emhum accession number AC005549 3 September 1998 BIRREN, B. ET AL.: "Homo sapiens chromosome 17, clone hRPK.215_E_13, complete sequence" XP002161329
- UEDA, A. ET AL.: "Transcriptional Regulation of The Human Monocyte Chemoattractant Protein-1 Gene" JOURNAL OF BIOLOGICAL CHEMISTRY, vol. 272, no. 49, 5 December 1997 (1997-12-05), pages 31092-31099, XP002161315
- WAGNER, K. ET AL.: "Mapping and functional characterization of DNase I hypersensitive sites in the mouse MCP-1 locus" KIDNEY INTERNATIONAL, vol. 50, no. 5, November 1996 (1996-11), page 1815 XP000926180
- UEDA, A. ET AL.: "NF-kappaB and Sp1 Regulate Transcription of the Human Monocyte Chemoattractant Protein-1 Gene" JOURNAL OF IMMUNOLOGY, vol. 153, no. 5, 1 September 1994 (1994-09-01), pages 2052-2063, XP000926179
- SOTO, U. ET AL.: "Conversion of HPV 18 positive non-tumorigenic HeLa-fibroblast hybrids to invasive growth involves loss of TNF-alpha mediated repression of viral transcription and modification of AP-1 transcription complex" ONCOGENE, vol. 18, no. 21, 27 May 1999 (1999-05-27), pages 3187-3198, XP000926178
- SHYY, Y.J. ET AL.: "Fluid shear stress induces a biphasic response of human monocyte chemotactic protein 1 gene expression in vascular endothelium" PROCEEDINGS OF THE NATIONAL ACADEMY OF SCIENCES OF USA, vol. 91, no. 11, 24 May 1994 (1994-05-24), pages 4678-4682, XP002161316
- FINZER, P. ET AL.: "Differential transcriptional regulation of the monocyte-chemoattractant protein-1 (MCP-1) gene in tumorigenic and non-tumorigenic HPV 18 positive cells: The role of the chromatin structure and AP-1 composition" ONCOGENE, vol. 19, no. 29, 6 July 2000 (2000-07-06), pages 3235-3244, XP000926181
- FRETER R.R. ET AL: 'A novel 7-nucleotide motif located in 3' untranslated sequences of the immediate-early gene set mediates platelet-derived growth factor induction of the JE gene' MOLECULAR AND CELLULAR BIOLOGY vol. 12, no. 12, December 1992,
- DATABASE EMBL [Online] HSJEPR, 03 November 1992 SCHWARZ E.: 'H. sapiens gene for JE protein, exons 3 and 4' Retrieved from EBI Database accession no. X60001

### SEQUENCE LISTING

<110> Deutsches Krebsforschungszentrum
<120> Novel regulatory sequences of the MCP-1 gene
<130> K 2817EU
<140> EP 00114560.6
   <141> 2000-07-06
<160> 12
<170> PatentIn Ver. 2.1
<210> 1
   <211> 600
   <212> DNA
   <213> Homo sapiens
<400> 1
   taggaaaatt ataggatcat taagaaagga gaaggaagag tgggagcaaa tacctggagg 60
   tagaaatggt gatgatgtgt acatcaagca gggagaaaac caatgaacca gatgcgaatt 120
   cgggcccaca ccaatgtcaa gggatgacaa ttagaaagga aggttgagtc aagggatttg 180
   aatgttaggg tgaaaagtta ctactcaact ctgtaggtta aaaggaaacg ttgagaatct 240
   tcagtccaat gaggagggat gtgccatgtt tagagattca gagataagtt tcaggaaatg 300
   taacttatag attttataca tacacagaga aatacggact agtgagaagc tattgccatg 360
   gtccaagcaa gagatgatga aggcctaaat atggagccaa agaggcagca atgaagaatg 420
   agccatgcag ggtgaaatgc tgcatgttgt aaatggagga gaaagacctg tgacttcaga 480
   tatgaaaacc tcatcttcaa cccacatttt aagggggcag cttccctgaa accagaatgt 540
   gtttccctcc attactatac ccccatccca atctcaggca cctggaatca tccatttaaa 600
<210> 2
   <211> 200
   <212> DNA
   <213> Homo sapiens
<400> 2
   tgcagctaac ttattttccc ctagctttcc ccagacacct tgttttattt tattataatg 60
   aattttgttt gttgatgtga aacattatgc cttaagtaat gttaattctt atttaagtta 120
   ttgatgtttt aagtttatct ttcatggtac tagtgttttt tagatacaga gacttgggga 180
   aattgctttt cctcttgtac                                             200
<210> 3
   <211> 150
   <212> DNA
   <213> Homo sapiens
<400> 3
   caaagatcac attctagctc tgaggtatag gcagaagcac tgggatttaa tgagctcttt 60
   ctcttctcct gcctgccttt tgctttttcc tcatgactct tttctgctct taagatcaga 120
   ataatccagt tcatcctaaa atgctttttc                                  150
<210> 4
   <211> 250
   <212> DNA
   <213> Homo sapiens
<400> 4
   aggcttctat gatgctacta ttctgcattt gaatgagcaa atggatttaa tgcattgtca 60
   gggagccggc caaagcttga gagctccttc ctggctggga ggccccttgg aatgtggcct 120
   gaaggtaagc tggcagcgag cctgacatgc tttcatctag tttcctcgct tccttccttt 180
   tctgcagttt tcgcttcaca gaaagcagaa tccttaaaaa taaccctctt agttcacatc 240
   tgtggtcagt                                                        250
<210> 5
   <211> 300
   <212> DNA
   <213> Homo sapiens
<400> 5
   aaggaggagg cagtgggcta ggagaatcga gagatcagaa ttttaaactc agcccagcca 60
   ttaacatgcc tcaagtactc ctatcatatt tgtaagagac aacagttcac tgaaatgaat 120
   tctaaggtct ttgggttttt atcagtgtgc ttctgtagtt tctgaggaaa tctaaggcac 180
   aactgaggaa tgaagtcagg ctttccaatt cccgaaatac tcctccactg cttactcatg 240
   tcccttggaa attaagaagg aagccaggag catagctgcc ataaccaggg atgaacttct 300
<210> 6
   <211> 300
   <212> DNA
   <213> Homo sapiens
<400> 6
   aaaatataaa aattagccag gcgtgatgtc atgtgcctgt agtcccagct actcgggagg 60
   ctgaggcagg agaacctctt gaatccagga ggcgcaggtt gcagtgagca gagatagtgc 120
   cactgcactc cagcctgggt gacagagtga gactctgtct caaaaaaata aaataaaata 180
   aaaaatgcag actgtgattc agcaggtctg ggttgaagcc cagaactctc tgataaattc 240
   aatggcactt aactacttgg aggtcatgga tgcctttgct aatctaatag aagctactga 300
<210> 7
   <211> 650
   <212> DNA
   <213> Homo sapiens
<400> 7
   ggcttgtgcc gagatgttcc cagcacagcc ccatgtgaga gctccctggc tccgggccca   60
   gtatctggaa tgcaggctcc agccaaatgc attctcttct acgggatctg ggaacttcca  120
   aagctgcctc ctcagagtgg gaatttccac tcacttctct cacgccagca ctgacctccc  180
   agcgggggag ggcatctttt cttgacagag cagaagtggg aggcagacag ctgtcacttt  240
   ccagaagact ttcttttctg attcataccc ttcaccttcc ctgtgtttac tgtctgatat  300
   atgcaaaggc caagtcactt tccagagatg acaactcctt cctgaagtag agacatgctt  360
   ccaacactca gaagcctatg tgaacactca gccagcaaag ctggaagttt ttctctgtga  420
   ccatgggcta attggtctcc ttctctggat tgtggcttat cagataaaaa caagtgagtc  480
   atgccacagg atgtctataa gcccattgat tctgggattc tatgagtgat gctgatatga  540
   ctaagccagg agagacttat ttaaagatct cagcatcttt cagcttgtta acctagagaa  600
   aacccgaagc atgactggat tataaaggga aattgaatgc ggtccaccaa             650
<210> 8
   <211> 20
   <212> DNA
   <213> Artificial Sequence
<220>
   <223> Description of the Artificial Sequence: Part of 3'-DHSR
<400> 8
   ggaaggttga gtcaaggatt                                             20
<210> 9
   <211> 21
   <212> DNA
   <213> Artificial Sequence
<220>
   <223> Description of the Artificial Sequence: Oligonucleotide
<400> 9
   gataaggtga ctcagaaaag g 21
<210> 10
   <211> 21
   <212> DNA
   <213> Artificial Sequence
<220>
   <223> Description of the Artificial Sequence: Oligonucleotide
<400> 10
   ggaaggttga gtcaagggat t 21
<210> 11
   <211> 21
   <212> DNA
   <213> Artificial Sequence
<220>
   <223> Description of the Artificial Sequence: Oligonucleotide
<400> 11
   cgcttgatga ctcagccgga a 21
<210> 12
   <211> 25
   <212> DNA
   <213> Artificial Sequence
<220>
   <223> Description of the Artificial Sequence: Oligonucleotide
<400> 12
   ttttggattg aagccaatat gataa 25

## Description

The present invention relates to a nucleic acid molecule comprising (a) a nucleic acid sequence encoding the monocyte-chemoattractant-protein-1 (MCP-1) or a protein having the biological activity of the monocyte-chemoattractant-protein-1 (MCP-1); and (b) a hypersensitive region.

The monocyte-chemoattractant-protein-1 (MCP-1) triggers the infiltration and activation of cells of the monocyte-macrophage lineage, representing known producers of growth-modulatory cytokines. Synthesis of cytokines can be considered an important regulatory loop in the immunological surveillance, since TNF-α or TGF-β can negatively interfere with the expression of high risk human papillomaviruses (HPV) types, the causative agents of cancer of the cervix uteri. Besides disturbance of a functional T-cell surveillance, dys-regulation of chemokine expression may represent another important counter-selection event during the multi-step progression to cervical cancer. Consistent with this anticipation was the recent finding that MCP-1 expression indeed gradually disappears in different stages of premalignant cervical intraepithelial neoplasias, a process, which may explain the severe reduction of intraepithelial macrophages and Langerhans' cells. It is also known that MCP-1 does not only play a critical role during tumor progression but also as regards atherosclerosis. However, due to the fact that so far the knowledge about the complex regulation of MCP-1 gene expression and regulatory regions involved was Wagner et al. (Kidney International vol. 50, no. 5 (1996), page 1815) reports the identification of DNAse I hypersensitive sites in the 3'and 5'regions of the mouse MCP-1 gene.
Schwarz E (EMBL Database ID HSJEPR) discloses the coding sequence of exons 3 and 4 of the human MCP-1 and a part of its 3'untranslated region.
Freter et al. (Molecular and Cellular Biology (1992), vol. 12, no. 12, pages 5288-5300) describes the analysis of 1.9kb of the 3'untranslated region of the murine JE gene (a synonym for MCP-1) and the detection of subreaions which regulate serum induction and response to PDGF IL-1 and double-stranded RNA. A 7-mer regulating 3'element was identified.

The technical problem underlying the present invention is to provide means for searching active substances for the treatment of diseases associated with an abnormal MCP-1 expression, e.g. atherosclerosis and cancer.

The solution to said technical problem is achieved by providing the embodiments characterized in the claims.

It has been, surprisingly, found that the expression of MCP-1 is dependent on a variety of cis-acting sequences which were so far not known. In the experiments leading to the present invention it could be shown that the expression of MCP-1 is closely linked with a non-tumorigenic phenotype in somatic cell hybrids made between the human papilloma virus type 18 (HPV 18) positive cervical carcinoma cell line HeLa and normal human fibroblasts. In contrast, MCP-1 transcription is absent in tumorigenic segregants derived from the same hybrids or in parental HeLa cells. Selectivity of MCP-1 transcription, which is regulated at the level of initiation of transcription, is mainly based on differences in the location and extension of DNAse I-hypersensitive regions (DHSR) at both ends of the gene or S1-hypersensitive sites (SHS). DNA sequencing showed that the hypersensitive sites often coincide with binding sites for transcription factors, like an AP-1 site, SP1 site, NF-IL6 site or NF kappa B site. Analyses of AP-1 composition revealed that MCP-1 is only expressed in those cells where *jun*-family members were mainly heterodimerized with the *fos*-related protein *fra-1*. In contrast, in tumorigenic cells the 1:1 ratio between jun and *fra-1* is disturbed and the MCP-1 gene is no longer expressed. Hence, alterations in the heterodimerization pattern of AP-1 and its selective accessibility to opened chromatin may represent a novel regulatory pathway in the regulation of chemokines in malignant and non-malignant HPV-positive cells.

The present invention relates to a nucleic acid molecule comprising
(a) a nucleic acid sequence encoding the monocyte-chemoattractant-protein-1 (MCP-1) or a protein having at least 80% amino sequence identity to the monocyte-chemoattractant-protein-1 (MCP-1) encoded by the EMBL clone Y 18933; and
(b) a 3'-DHSR comprising a nucleic acid molecule which is located 2430 bp to 3019 bp downstream of the transcriptional start site of the MCP-1 gene and has at least 80% identity with the nucleic acid sequence of SEQ ID NO:1, wherein said 3' DNAse I-hypersensitive region comprises the nucleic acid sequence TGAGTCA.

As used herein, the term "a nucleic acid molecule encoding the monocyte-chemoattractant-protein-1 (MCP-1) or a protein having at least 80% amino acid sequence identiy to the monocyte-chemoattractant-protein-1 (MCP-1)" relates to the published sequence of the MCP-1 gene, e.g. the EMBL database sequence (accession number: Y 18933), the sequence published by Rollins et al., Mol. Cell. Biol. 9 (1989), 4687-4695) or a related sequence which still encodes a protein having at least 80% amino acid sequence identity to said MCP-1.
These related sequences comprise sequences which hybridize to the above published MCP-1 nucleic acid sequences. As used herein, the term "hybridize" has the meaning of hybridization under conventional hybridization conditions, preferably under stringent conditions as described, for example, in Sambrook et al., Molecular Cloning, A Laboratory Manual, 2^{nd} edition (1989) Cold Spring Harbor Laboratory Press, Cold Spring Harbor, NY. Also contemplated are nucleic acid sequences that hybridize to the MCP-1 nucleic acid sequences at lower stringency hybridization conditions. Changes in the stringency of hybridization and signal detection are primarily accomplished through the manipulation of formamide concentration (lower percentages of formamide result in lowered stringency), salt conditions, or temperature. For example, lower stringency conditions include an overnight incubation at 37°C in a solution comprising 6X SSPE (20 x SSPE = 3M NaCl; 0.2M NaH₂PO4; 0.02M EDTA, pH 7.4), 0.5% SDS, 30% formamide, 100 µg/ml salmon sperm blocking DNA, followed by washes at 50°C with 1 x SSPE, 0.1% SDS. In addition, to achieve even lower stringency, washes performed following stringent hybridization can be done at higher salt concentrations (e.g. 5 x SSC). Variations in the above conditions may be accomplished through the inclusion and/or substitution of alternate blocking reagents used to suppress background in hybridization experiments. The inclusion of specific blocking reagents may require modification of the hybridization conditions described above, due to problems with compatibility.

These related nucleic acid sequences also include sequences that are degenerate as a result of the genetic code or fragments, derivatives and allelic variants of the nucleic acid molecules described above encoding a MCP-1 protein or a protein having MCP-1 activity. "Fragments" are understood to be parts of the nucleic acid sequences that are long enough to encode one of the described proteins. The term "derivative" in this context means that the sequences of these molecules differ from the sequences of the nucleic acid sequences described above at one or several positions but have a high level of homology to these sequences. Homology hereby means a sequence identity of at least 40%, in particular an identity of at least 60%, preferably of more than 80% and particularly preferred of more than 90%. These proteins encoded by the nucleic acid molecules have a sequence identity to the amino acid sequence encoded by the EMBL clone (Y 18933) of at least 80%, preferably of 85% and particularly preferred of more than 90%, 95%, 97% and 99%. The deviations to the above-described nucleic acid sequences may have been produced by deletion, substitution, insertion or recombination.

The nucleic acid sequences that are homologous to the above-described sequences and that represent derivatives of these sequences usually are variations of these molecules that represent modifications having the same biological function. They can be naturally occurring variations, for example sequences from other organisms, or mutations that can either occur naturally or that have been introduced by specific mutagenesis. Furthermore, the variations can be synthetically produced sequences. The allelic variants can be either naturally occurring variants or synthetically produced variants or variants produced by recombinant DNA processes.

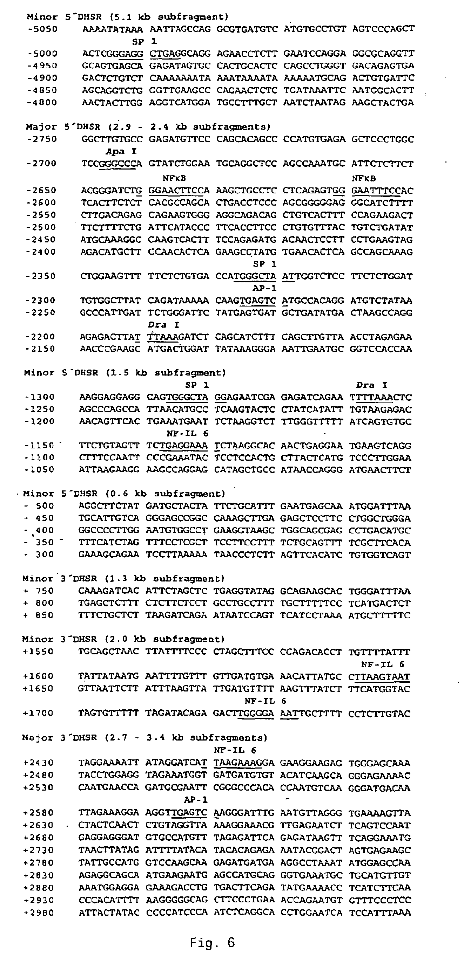

The hypersensitivity regions as identified are depicted in Figure 6. The hypersensitive region according to the invention is the 3'-DHSR comprising the nucleic acid sequences from position + 2430 bp to + 3019 bp as shown in Fig. 6. In a more preferred embodiment of the present invention, the 3'-DHSR comprises the nucleic acid sequence GGAAGGTTGAGTCAAGGATT. In the present invention, the 3'-DHSR comprises the nucleic acid molecule TGAGTCA. As used herein, the 3'-DHSR sequences also comprise sequences containing alterations compared to the particular sequences shown in the figures or to the published sequences which do not lead to a loss of the function as cis-acting element, e.g. as regards gene expression.

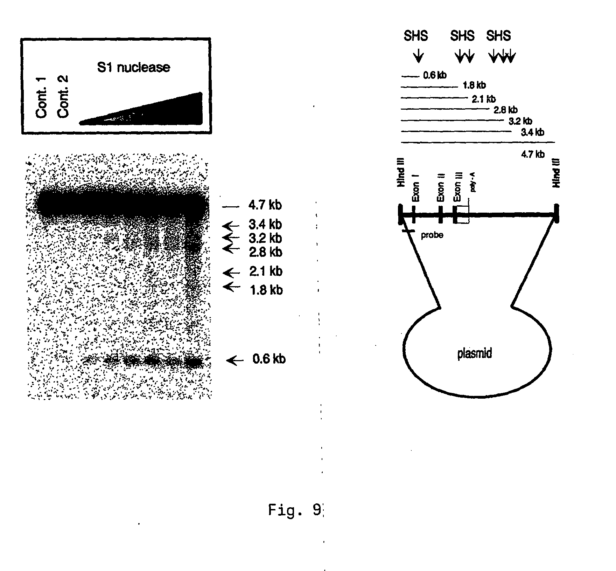

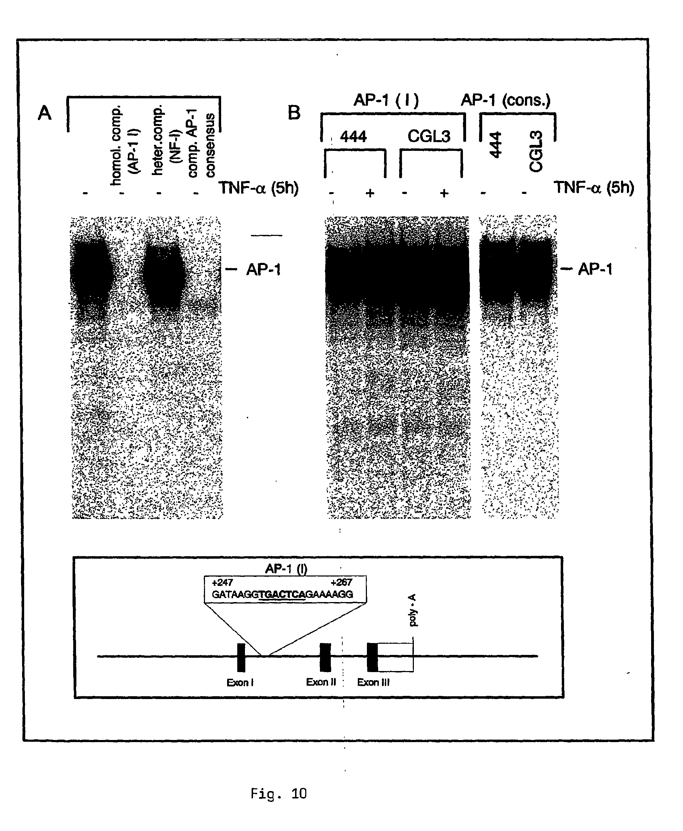

The S1 hypersensitivity region (SHS) in the 1st intron (0.6 kb) of MCP-1 has been identified by S1 nuclease mapping (c.f. Figs. 9 and 10). This SHS contains an AP-1 site which has been verified by band-shift analysis and TNF induction.

The present invention also relates to a nucleic acid molecule wherein the hypersensitive sites contain mutations resulting in a modified DNAse I hypersensitivity and S1 hypersensitivity, respectively, or altered interaction with transcription factors, in particular altered interaction with AP-1. Generally, by means of conventional molecular biological processes it is possible (see, e.g., Sambrook et al., 1989, Molecular Cloning, A Laboratory Manual, 2^{nd} edition Cold Spring Harbor Laboratory Press, Cold Spring Harbor, NY) to introduce different mutations. One possibility is the production of deletion mutants in which nucleic acid molecules are produced by continuous deletions from the 5'- or 3'-terminus of the DNA sequence. Another possibility is the introduction of single-point mutation at positions where a modification influences the interaction with influences the interaction with transcription factors, e.g. AP-1. The identity of the mutated sequence with the original sequence is at least 80% and particularly preferred more than 90%.

For the manipulation in prokaryotic cells by means of genetic engineering the nucleic acid molecules of the invention or parts of these molecules can be introduced into plasmids allowing a mutagenesis or a modification of a sequence by recombination of DNA sequences. By means of conventional methods (cf. Sambrook et al., 1989, Molecular Cloning: A Laboratory Manual, 2^{nd} edition, Cold Spring Harbor Laboratory Press, NY, USA) bases can be exchanged and natural or synthetic sequences can be added. In order to link the DNA fragments with each other adapters or linkers can be added to the fragments. Furthermore, manipulations can be performed that provide suitable cleavage sites or that remove superfluous DNA or cleavage sites. If insertions, deletions or substitutions are possible, in vitro mutagenesis, primer repair, restriction or ligation can be performed. As analysis method usually sequence analysis, restriction analysis and other biochemical or molecular biological methods are used.

The invention furthermore relates to vectors containing the nucleic acid molecules of the invention. Preferably, they are plasmids, cosmids, viruses, bacteriophages and other vectors usually used in the field of genetic engineering. Vectors suitable for use in the present invention include, but are not limited to T7-based expression vectors for expression in bacteria, the pMSXND expression vector for expression in mammalian cells and baculovirus-derived vectors for expression in insect cells. Preferably, the nucleic acid molecule of the invention is operatively linked to the regulatory elements in the recombinant vector of the invention that guarantee the transcription and synthesis of an RNA in prokaryotic and/or eukaryotic cells that can be translated. The nucleotide sequence to be transcribed can be operably linked to a promotor like a T7, metallothionein I or polyhedrin promotor.

In a further embodiment, the present invention relates to recombinant host cells transiently or stably containing the nucleic acid molecules or vectors of the invention.

Preferably, these cells are prokaryotic or eukaryotic cells, for example mammalian cells, bacterial cells, insect cells or yeast cells.

The delivery of nucleic acid molecules encoding proteins useful for regulating MCP-1 expression or of nucleic acid molecules with mutated hypersensitivity region sequences can be achieved by direct application or, preferably, by using a recombinant expression vector such as a chimeric virus containing these compounds or a colloidal dispersion system. By using appropriate vectors the mutated sequences can be inserted into the genome and, e.g., replace the original sequences. By delivering these nucleic acids to the desired target, the intracellular expression of MCP-1 and, thus, the level of MCP-1 can, e.g., be decreased resulting in the inhibition of the negative effects of MCP-1, e.g. as regards atherosclerosis. On the orther hand, by using this approach an increased MCP-1 expression (or re-expression of MCP-1) can be achieved, e.g. for the treatment or prevention of tumors, e.g. cervical carcinoma.

The above nucleic acid sequences can be administered directly to the target site, e.g., by ballistic delivery, as a colloidal dispersion system or by catheter to a site in artery. The colloidal dispersion systems which can be used for delivery of the above nucleic acids include macromolecule complexes, nanocapsules, microspheres, beads and lipid-based systems including oil-in-water emulsions, (mixed) micelles, liposomes and lipoplexes. The preferred colloidal system is a liposome. The composition of the liposome is usually a combination of phospholipids and steroids, especially cholesterol. The skilled person is in a position to select such liposomes which are suitable for the delivery of the desired nucleic acid molecule. Organ-specific or cell-specific liposomes can be used, e.g. in order to achieve delivery only to the desired tissue. The targeting of liposomes can be carried out by the person skilled in the art by applying commonly known methods. This targeting includes passive targeting (utilizing the natural tendency of the liposomes to distribute to cells of the RES in organs which contain sinusoidal capillaries) or active targeting (for example by coupling the liposome to a specific ligand, e.g., an antibody, a receptor, sugar, glycolipid, protein etc., by well known methods). In the present invention monoclonal antibodies are preferably used to target liposomes to specific tumors via specific cell-surface ligands.

Preferred recombinant vectors useful for gene therapy are viral vectors, e.g. adenovirus, herpes virus, vaccinia, or, more preferably, an RNA virus such as a retrovirus. Even more preferably, the retroviral vector is a derivative of a murine or avian retrovirus. Examples of such retroviral vectors which can be used in the present invention are: Moloney murine leukemia virus (MoMuLV), Harvey murine sarcoma virus (HaMuSV), murine mammary tumor virus (MuMTV) and Rous sarcoma virus (RSV). Most preferably, a non-human primate retroviral vector is employed, such as the gibbon ape leukemia virus (GaLV), providing a broader host range compared to murine vectors. Since recombinant retroviruses are defective, assistance is required in order to produce infectious particles. Such assistance can be provided, e.g., by using helper cell lines that contain plasmids encoding all of the structural genes of the retrovirus under the control of regulatory sequences within the LTR. Suitable helper cell lines are well known to those skilled in the art. Said vectors can additionally contain a gene encoding a selectable marker so that the transduced cells can be identified. Moreover, the retroviral vectors can be modified in such a way that they become target specific. This can be achieved, e.g., by inserting a polynucleotid encoding a sugar, a glycolipid, or a protein, preferably an antibody. Those skilled in the art know additional methods for generating target specific vectors. Further suitable vectors and methods for in vitro- or in vivo-gene therapy are described in the literature and are known to the persons skilled in the art; see, e.g., WO 94/29469 or WO 97/00957.

### Brief description of the drawings

### Figure 1:

**Panel A:** Selective induction and time course of MCP-1 expression. 5 x 10⁵ cells were incubated in the presence of 250 U/ml of TNF-α for 5 and 18 hours, respectively. 5 *µ*g RNA of each HeLa cells, non-tumorigenic HeLa-fibroblast hybrids ("444") and their tumorigenic segregants ("CGL3") was separated in a 1% agarose gel. After blotting, the filter was consecutively hybridized with cDNA probes encoding the MCP-1 and the β-actin gene. (-): untreated control cells. The positions of the 18 and 28S rRNAs are indicated. **Panel B:** cell morphology of HeLa-, "444"- and "CGL3" cells either untreated (A, C, E) or after simultaneous incubation with TNF-α (500 U/ml) and cycloheximide (10 *µ*g/ml) for 4 hours at 37°C. Phase-contrast microscopy (Magnification: 100 x). **Panel C:** degradation and re-synthesis of IκB-α after treatment of HeLa-, "444"-, and "CGL3"-cells with 250 U/ml TNF-α for 30 minutes (min) or 5 hours (h). 30 *µ*g total cellular protein was separated in a 12% SDS-PAGE gel for Western blot analysis. After electrotransfer, the filter was probed with a polyclonal antibody directed against IκB-α. To confirm equal loading, an identical filter was incubated with a β-actin specific antibody.

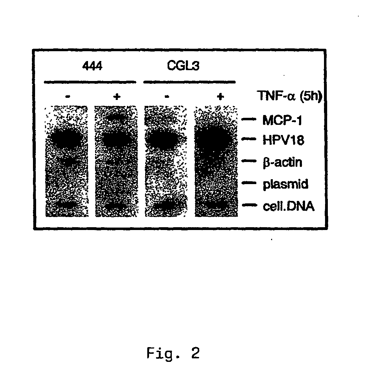

### Figure 2: Nuclear run-on analysis

Ongoing transcription from TNF-α treated (250 U/ml, for 5 hours) and control nuclei was examined by hybridizing equal counts of ³²P-UTP nascent RNA to nitrocellulose strips carrying the same amount of heat-denaturated DNA probes (2.5 ig of MCP-1, HPV 18, β-actin and p"Bluescript" plasmid DNA). To confirm identical hybridization conditions, 50 ng of total cellular DNA was loaded as an intemal reference. (-): untreated cells; (+) cells incubated with 250 U/ml of TNF-α.

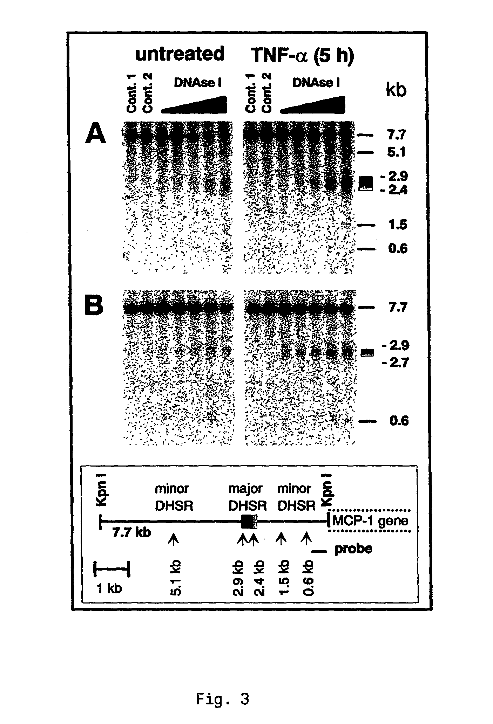

### Figure 3: DNAse I mapping analysis of the 5'-regulatory region

"444" cells (panel A) and "CGL3" cells (panel B) were treated for 5 hours with TNF-α (250 U/ml). Isotonic nuclei equivalent to 100 *µ*g of total DNA from either untreated or TNF-α treated cells were subsequently digested with increasing amounts of DNAse I (1.0; 2.0; 3.0; 4.0; and 5.0 units of DNAse I/100 *µ*l volume) for 5 minutes at 37°C. After extraction, the DNAs were cleaved with Kpn I, separated on 1% agarose gels and hybridized with a cloned PCR fragment located adjacent to Kpn I (pos. -539 to +52 relative to the transcriptional start site defined by Ueda and coworkers (Ueda et al., J. Immunol. 153 (1994), 2052-2063) for indirect end-labeling. The length of the subbands generated after DNAse I/Kpn I digestion is indicated in kb. "Cont 1": nuclei lysed immediately after preparation: "Cont 2": DNA from nuclei incubated in digestion buffer without DNAse I. The schematic presentation below shows the location of the major and minor DNAse I hypersensitive regions (DHSR) relative to the Kpn I site (pos. +211/+216) in both cell lines (indicated by an arrow). The length of the parental 7.7 kb Kpn I/Kpn I fragment and the position of hybridization probe ("probe") are indicated.

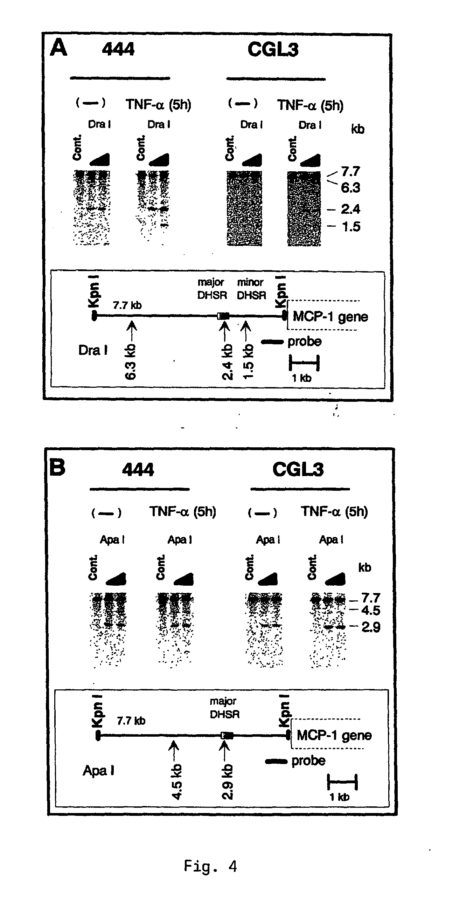

### Figure 4: Accessibility of the DHSRs to restriction endonucleases in native chromatin

**Panel A:** isotonic nuclei corresponding to an amount of 100 µg total cellular DNA from untreated (-) and TNF-α treated (250 U/ml, for 5 hours) "444"- and "CGL3"-Cells were digested with increasing amounts of Dra I (25 and 50 units for 15 minutes at 37°C). After restriction enzyme digestion, the DNAs were processed as described in Fig. 3. "Cont": DNA from nuclei incubated in digestion buffer for 15 minutes at 37°C without any enzyme. The schematic presentation shows the length and the derivation of the subbands generated after Dra I/Kpn I cleavage (for details, see Fig. 3). **Panel B:** same as panel A, only after treatment of the nuclei with Apa I restriction endonuclease.

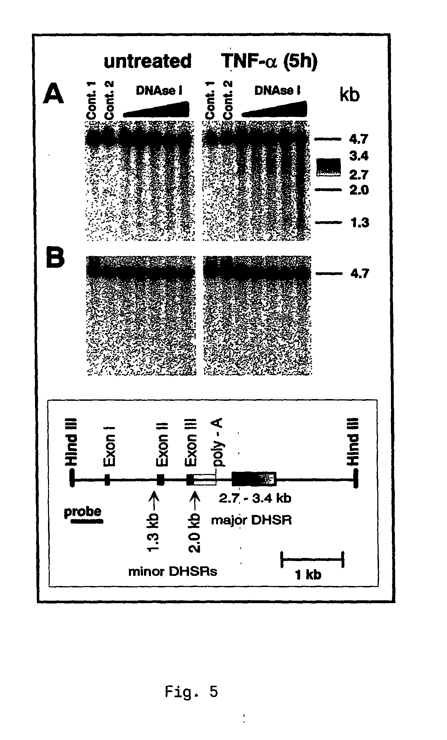

### Figure 5: DNAse I mapping analysis of the 3'-regulatory region

"444" cells **(panel A)** and "CGL3" cells **(panel B)** were treated with TNF-α as descibed in Fig. 3. Nuclei corresponding to 100 *µ*g of total DNA obtained from untreated and cytokine treated cells were digested with increasing amounts of DNAse I (1.0; 2.0, 3.0, 4.0, and 5.0 units of DNAse I/100 *µ*l volume) for 5 minutes at 37°C. After lysis, the purified DNAs were cleaved with Hind III, separated on 1% agarose gels and hybridized with a cloned PCR fragment (pos. -539 to +52 relative to the transcriptional start site) located adjacent to the Hind III site for indirect end-labeling. The length of the subbands generated after DNAse I/Hind III digestion is indicated in kb. "Cont 1": nuclei immediately after preparation; "Cont 2": DNA from nuclei incubated in digestion buffer without adding enzyme. The schematic presentation below shows the location (indicated by an arrow) of the major and minor DNAse I hypersensitive regions (DHSR) relative to the Hind III site (pos -428/-423, based on the transcriptional start site). The length of the parental 4.7 kb Hind III/Hind III fragment, the position of hybridization probe ("probe"), the three exons ("Exon I-III") and the location of the chemokine specific polyadenylation signal ("poly-A") are indicated.

### Figure 6: Nucleotide sequence analysis of the 5'- and 3' regulatory region of the human MCP-I gene covered by DNAse I hypersensitive regions (DHSRs) in native chromatin

Nucleotide numbering was done relative to the transcriptional start site (Ueda et al., 1994, J Immunol. 153, p. 2052-2063). Some DHSRs coincide with a DNA fragment shown to harbor functional binding sites for the transcriptional factors, AP-1 and NF-κB (see nucleotide stretch ranging from pos. -2799 to -300). The positions of additional putative binding sequences for the transcription factors AP-1, NF-1L-6, Sp1 and the restriction endonucleases Apa I and Dra I are indicated. The complete nucleotide sequence (11793 bp) of the whole MCP-1 gene locus is available at the EMBL nucleotide sequence data base (accession number: Y 18933).

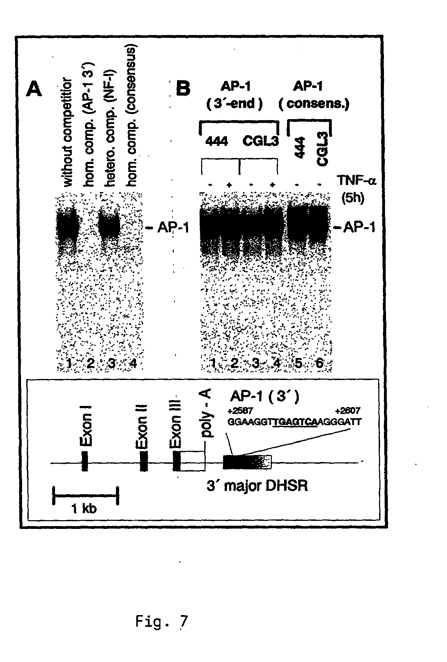

### Figure 7: Band shift analysis of the 3'AP-1 site within the major DHSR

**Panel A:** 2.5 µg "444" cell extract was incubated with a ³²P-labeled oligonucleotide from pos. +2587/+2607 (referred as AP-1 (3'), see schematic presentation depicted below). Lane 1: without competition; lane 2: addition of a 100-fold molar excess of unlabeled homologous oligonueleotides ("hom. comp. AP-1 3'"); lane 3: addition of a 100-fold molar excess of an oligonucleotide harbouring a recognition site for the transcription factor NF-1("hetero. comp. NF-1"); lane 4: competition with a 100-fold excess of an oligonucleotide containing an AP-1 site of the human collagenase gene ("hom. comp. consensus"). **Panel B:** (lanes 1-4) band-shift analysis using the AP-1 3'-oligonucleotide ("AP-1 3'-end") after incubation with nuclear extracts derived from control (-) and TNF-α treated (+) "444"- and "CGL3" cells. Lanes 5 and 6: band-shift pattern of an AP-1 oligonucleotide from the human collagenase gene ("AP-1 consens.") using either "444" or "CGL3" nuclear extracts. Cells were treated with TNF-α (250 U/ml) for 5 hours (5h). The position of the AP-1 specific DNA-protein complex is indicated. The schematic presentation below shows the location and sequence of the 3'-AP-1 site within the 3'-major DHSR relative to the genome organization of the MCP-1 gene.

### Figure 8:

**Panel A:** Supershift-analysis of the AP-1 complex in "444" cells and "CGL3" cells. ³²P-labeled AP-1 specific oligonucleotides derived from the 3'-DHSR (referred as "AP-1 3'-end") were incubated with nuclear extracts from "444"- and "CGL3" cells either in the absence (lanes 1 and 5) or in the presence of specific antibodies raised against *c-jun* (lanes 2 and 6), *c-fos* (lanes 3 and 7) or *fra-1* (lanes 4 and 8). The arrowhead marks the position of the retarded "super-shift" complexes. **Panel B:** selective induction of the human type I collagenase gene in "444" cells after TNF-α treatment (250 U/ml) for 30 minutes (30'), 4 and 8 hours (4h, 8h). RNA was extracted and monitored by Northern blot analysis. The same filter was consecutively hybridized with a cDNA probe homologous to the human collagenase and the β-actin. gene. The ethidium bromide stained gel shows the integrity and the position of the 28S and 1 M rRNA. (-): untreated control cells.

### Figure 9:

The HindIII/HindIII-fragment (c.f. Fig. 5) was cloned into pBluescript, purified by CsCl centifiugation and digested with increasing amounts of S1 nuclease. The DNas were separated on 1 % agarose gels and hybridized with a cloned PCR fragment (position -539 to +52 relative to the transcriptional start site) located adjacent to the HindIII site for indirect-end-labeling. The length of the subbands generated after S1 nuclease digestion is indicated in kb. "Cont1": untreated plasmids; "Cont.2": DNA incubated in digestion buffer without adding enzyme. The schematic presentation below shows the location (indicated by an arrow) of the S1 hypersensitive sites (SHS) relative to the HindIII site (position -428/-423). The length of the parental 4.7 kb HindIII/HindIII fragment, the position of the hybridization probe ("probe"), the three exons (Exons I-III) and the localisation of the chemokine specific polyadenylation site (polyA) are indicated.

### Figure 10:

Band-shift analysis of the AP-1 site located within the 1. intron of the MCP-1 gene. A: 2,5 µg "444" extract was incubated with a ³²P-labeled oligonucleotide from position +247/+267 (GATAAGGTGACTCAGAAAAGG, refered as AP-1(I), see schematic presentation depicted below). Lane 1: without competition, lane 2: addition of a 100-fold molar excess of unlabeled homologous oligonucleotides ("homol. comp.(AP-1(I)); lane 3: addition of a 100-fold molar excess of an oligonucleotide haboring a recognition site for the transcription factor NF-1 ("heter. comp."); lane 4: competition with a 100-fold excess of an oligonucleotide containing an AP-1 site of the human collagenase gene ("comp. AP-1 consensus")
B: First four lanes represent band-shift analysis using the AP-1 (I) oligonucleotide (AP-1 (I)) after incubation with nuclear extracts derived from control (-) and TNF-α treated (+) "444"- and "CGL3" cells. Right two lanes represent band-shift pattern of an AP-1 oligonucleotide from the human collagenase gene (AP-1 cons.) using either "444"- or "CGL3" nuclear extracts. Cells were treated with TNF-α (250 U/ml) for 5 h. The position of the AP-1 specific DNA protein complex is indicated. The schematic presentation below shows the location and the sequence of the AP-1 (I) site within the 1st intron relative to the genome organisation of the MCP-1 gene.

The following examples illustrate the invention.

### EXAMPLE 1: GENERAL METHODS

### (A) Cell lines

HPV 18-positive cervical carcinoma cells (HeLa), non-tumorigenic somatic cell hybrids made between HeLa cells and normal human fibroblasts ("444") (Stanbridge, Cancer Survey 3 (1984), 335-350), the corresponding tumorigenic segregants ("CGL3") (Stanbridge, Cancer Survey 3 (1984), 335-350) and the human glioblastoma cell line A172 (Tumorbank DKFZ, Heidelberg) were maintained in Dulbecco's modified Eagle's medium, supplemented with 10% fetal calf serum and 1% penicillin/streptomycin

### (B) Cytokines and reagents

For cytokine treatment, the cells were incubated with TNF-α (specific activity: 1 x 10⁸ units/mg) (Pharma Biotechnologie, Hannover, Germany) for different periods of time as described in the figure legends. Cycloheximide (Sigma, St. Louise, USA) was stored as a 10 mg/ml stock solution at -20°C.

### (C) RNA analysis

Total cellular RNA was extracted according to the guanidine-thiocyanate procedure (Chomczynski and Sacchi, Anal. Biochem. 162 (1987), 156-159). Approximately 5 *µ*g of total cellular RNA was separated on 1% agarose gels as described elsewere (Rösl et al., J. Virol. 68 (1994), 2142-2150).

### (D) Quantification of apoptosis

The rate of apoptosis was determined by a cell death detection ELISA^{PLUS} kit (Roche) as recommended by the manufacturer. The enrichment factor was calculated by setting the absorbance of the untreated cells as 1.

### (E) Isolation and DNA sequence analysis of the human MCP-1 gene

A chromosome 17 specific cosmid library (Resourcenzentrum Berlin; Zehetner, G. Lehrach, H. (1994), Nature 367, p. 489-491) was screened using the cDNA of MCP-1 as a probe. The sequence of the flanking genomic regions was finally determined using two plasmid clones harboring overlapping inserts of about 7.7 kb (a Kpn I/Kpn I-fragment for the 5'-region) and a 4.7 kb (Hind III/Hind III-fragment for intron/exon and the 3'-region) in size. DNA sequence analysis was done on both strands using chemically synthesized primers and fluorescent sequencing on an automatic sequencer (Model 377, Perkin-Elmer/Applied Biosystems (Foster City, USA) and the dye terminator method according to the manufacturers protocol ("ABI PRISM Big Dye Ready Reaction Terminator Cycle Sequencing Kit", Perkin-Elmer/Applied Biosystems). Putative binding sites for transcription factors were identified by the computer program "Transfac version 3.3" (Wingender et al., Nucl. Acids. Res. 25 (1997), 265-268). The complete 11793 bp sequence of the human MCP-1 gene locus encompassing the 5'- and 3'-regulatory regions as well as the introns is available at the EMBL nucleotide sequence data base (accession number: Y18933).

### (F) RNA and DNA probes

HPV 18 represents the unit-length of HPV 18 DNA cloned in pBR322 (Boshart et al., EMBO J. 3 (1984), 1151-1157). pHFAI (Gunning et al., Mol. Cell. Biol. 3 (1983), 787-795) containing an approximately full-length insert of the fibroblast β-actin gene was a generous gift from L. Kedes (Medical Center, Palo Alto, USA). The full length cDNA encoding the monocyte-chemoattractant-protein-1 (MCP-1) (Rollins et al., Mol. Cell. Biol. 9 (1989), 4687-4695) was obtained from the American Type Culture Collection (Kockville, MD, USA; ATCC 61364/61365, plasmid clone pGEM-hJE34, HGLM probe ID: p11696). The cDNA of the human type 1 collagenase gene (Offringa et al., Cell 62 (1990), 527-538) was kindly provided by P. Angel (Deutsches Krebsforschungszentrum). The PCR product encompassing parts of the MCP-1 promotor ranges from -539 to +52 (according to Ueda et al., 1994) was amplified and subcloned as described previously (Rösl et al., 1994).

### (G) SDS-PAGE and Western blotting

Cellular extracts (Dignam et al., Nucl. Acids Res. 11, (1983), 1475-1489) were separated in sodium dodecyl sulfate (SDS) 12% polyacrylamide gels (PAGE), electrotransferred to PVDF membranes (Immobilon-P, Millipore Corporation Bedford, MA, USA) and probed with polyclonal rabbit IκBα antibodies (epitope corresponding to the amino acids 297-317 mapping at the carboxy terminus, Santa Cruz Biotechnology, California, USA) as described recently (Soto et al., Oncogene 18 (1999), 3187-3198). Equal protein transfer and loading was routinously monitored by incubating identical filters with a monoclonal β-actin specific antibody (ICN Biomedicals, Ohio, USA).

### (H) Nuclear run-on assays

Nuclei from exponentially growing cells were prepared as described recently (Maehama et al., Int. J. Cancer 76 (1998), 639-646). Heat-denaturated linearized DNA fragments were fixed onto nitrocellulose filters using the "S+S minifold II slot apparatus" (Schleicher and Schuell, Dassel, Germany) and hybridized with equal amounts of ³²P-labelled RNA. After 48h, the filters were washed with 2 x SSC/0.1% SDS at 68°C.

### (I) Isolation of nuclei and nuclease digestion conditions for chromatin analysis

Nuclei from HeLa-, "CGL3"- and "444"-cells were prepared (Rösl et al., Mol. Carcinog. 2 (1989), 72-80) and digested as described in the figure legends. Southem blot analyses were done using standard protocols.

### (J) Electrophoretic mobility shift assays

To analyse protein/DNA interactions by electrophoretic mobility shift assays (EMSAs), the following oligonucleotides were used: the AP-1 binding site (5'-GGAAGGTTGAGTCAAGGGATT-3') located within the DNAse I hypersensitive site downstream of the MCP-1 gene (pos. +2587 / +2607), an AP-1 consensus sequence, 5'-CGCTTGATGACTCAGCCGGAA-3' (Lee et al., Cell 49 (1987), 741-752), an oligonuoleotide containing a recognition site for the nuclear factor 1 (NF-1) derived from the adenovirus origin, 5'-TTTTGGATTGAAGCCAATATGATAA-3' (Kenny and Hurwitz, J. Biol. Chem. 263 (1988), 9809-9817). The DNAs were synthesized using a phosphoramitide chemistry (Applied Biosystems synthesizer) and further purified by HPLC. Preparation of nuclear extracts, electrophoretic mobility shift and supershift assays were performed exactly as described (Soto et al., Oncogene 18 (1999), 3187-3198).

### EXAMPLE 2: Selective MCP-1 inducibility by TNF-α: demonstration of the integrity of the TNF-α signal transduction pathway in malignant and non-malignant HPV 18-positive cells

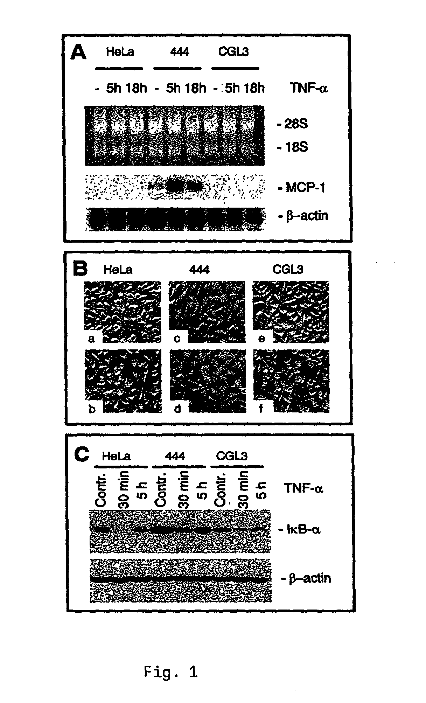

By monitoring the expression of the MCP-1 gene in HPV 18 positive cells differing in their potential to form tumors in nude mice, it was found that MCP-1 was transcribed and TNF-α indicible only in non-malignant hybrids made between HeLa cervical carcinoma cells and normal human fibroblasts (Fig. 1A, designated as "444"). In contrast, examination of either the tumorigenic segregants from the same hybrids (designated as "CGL3") or the parental tumorigenic HeLa cells showed the complete absence of detectable transcripts under the same experimental conditions. The intensity of the MCP-1 mRNA reached a maximum 5 hours after TNF-α addition and declined to almost baseline level 18 hours after TNF-α treatment. The absence of MCP-1 inducibility in the tumorigenic cells cannot be attributed to disturbances in the cytokine signal transduction pathway, since the simultaneous application of TNF-α and a protein synthesis inhibitor (cycloheximide) for 4 hours induced blebbing of the cellular membrane (Fig. 1 B), a morphological feature characteristic for TNF-α mediated apoptosis. The extend of apoptosis was quantified using a cell death detection ELISA kit which determines the amount of free histones within the cytoplasmic fraction after nuclear envelope breakdown and DNA fragmentation into discrete multiplicities of mononucleosomes (Fig. 1 C). The integrity of the TNF-α pathway was further confirmed by the rapid proteolysis (after 30 minutes) and delayed resynthesis (after 5 hours) of the inhibitory component IκB-α (Fig. 1 D), leading to the activation of the transcription factor NF-κB.

### EXAMPLE 3: MCP-1 mRNA expression is regulated at the level of initiation of transcription

In order to address the question of whether the restricted expression of MCP-1 and its TNF-α inducibility in non-tumorigenic cells was due to a transcriptional block, nuclear run-on experiments were carried out. Hybridization of equal counts of ³²P-UTP labeled total RNA to defined amounts of immobilized DNA showed the absence of MCP-1 expression in TNF-α treated tumorigenic "CGL3" cells, while the gene became strongly induced in "444" cells 5 hours after TNF-α addition (Fig. 2). The induction was selective, which was confirmed by a direct comparison with the degree of transcriptional activity of other reference genes such as HPV 18 or β-actin. Furthermore, loading a small amount of total cellular DNA on nitrocellulose strips demonstrated that the total ³²P-UTP incorporation of the nascent RNA was the same. These data indicate that TNF-α mediated MCP-1 induction is regulated at the level of initiation of transcription rather than by post-transcriptional mechanisms.

### EXAMPLE 4: Mapping of DNAse I hypersenvitive regions (DHSRs) at the 5'-and 3'-end of the MCP-1 gene in native chromatin: evidence for a novel TNF-α inducible DHSR at the 3'-end

In order to obtain more information on MCP-1 transcriptional regulation, the chromatin structure of the whole MCP-1 locus was examined. As shown in Figure 3A, there is one major DNAse I hypersensitive region (DHSR) in "444"-cells, whose maximum DNAse I cutting frequency was located approximately 2.4-2.9 kb upstream of the Kpn I site (Pos. +211/+216.relative to the transcriptional start site; see Ueda et al., 1994). In contrast, the major DHSR visible in "CGL3" cells has a smaller extension only ranging from 2.7-2.9 kb (Fig. 3B). In addition, the 5'-chromatin structure in "444"-cells revealed the presence of two minor DHSRs (see schematic presentation below): a distal and a proximal one, located 5.1 and 1.5 kb, respectively, upstream of the Kpn I site (for DNA sequence analysis, see Fig. 6).

Taking advantage that the restriction endonuclease Dra I is located not only within the minor distal DHSR (pos. -1259/-1254) but also at the 3'-boundary of the major "444"-DHSR, (pos. -2186/-2191, see also Fig. 6), incubation of isolated nuclei with Dra I provides additional evidence for the larger size and higher TNF-α mediated susceptibility of this chromatin stretch in non-malignant cells. As shown in Fig. 4A, digestion of "444" derived nuclei led to a fast and strong appearance of two Dra I-derived subfragments (2.4 and 1.5 kb, respectively), whose intensity were even pronounced after TNF-α application. Quantifying the resulting subbands relative to the intensity of the undigested parental 7.7 kb fragment showed that already at 15 minutes incubation with 25 units of Dra I was sufficient to cleave almost 45% of the chromatin (corresponding to 100 µg DNA). In contrast, the same region was much more resistant in untreated "CGL3" control cells (Fig. 4A). Accessibilily to Dra I was only weakly elevated by TNF-α (accumulation of the cleaved subbands increased from 6 to 12% relative to the parental fragment), unequivocally demonstrating a different degree of chromatin decondensation in both cell lines. Treating the same nuclei with Apa I (pos. -2692/-2697), a restriction endonuclease which is located directly within the major 5'-DHSRs in both cell lines, no differences in the temporal appearance and the intensity of the generated subbands could be observed (Fig. 4B).

Cytokine treatment led to a strong appearance of a prominent 3'-DHSR, which is located between 2.7-3.4 kb downstream of the Hind III recognition site (pos. -428/-423). Furthermore, several sensitive regions could be noticed (Fig. 5, see schematic overview). It should be stressed that major 3'-DHSR formation was not merely the consequence of MCP-1 transcription per se, because the chromatin of the untreated "444" control cells was resistant against DNAse I (Fig. 5, panel A, left), despite ongoing baseline MCP-1 expression was detectable (see Fig. 1, panel. A). Furthermore, TNF-α kinetics and subsequent DNAse I mapping analyses indicated that DHSR configuration even slightly preceded MCP-1 induction, suggesting that changes in the nucleosomal organization was a requisite rather than a consequence of TNF-α mediated MCP-1 transcription. Carrying out the same experimental approach for both the tumorigenic and segregants (Fig. 5B) or the parental HeLa cells, no 3'-DHSR could be detected. One can therefore conclude that the differences of MCP-1 transcription and TNF-α inducibility in tumorigenic and non-tumorigenic HPV-positive cells is obviously mediated by a concerted action of at least two different regulatory regions. One is located at the 5'-end, being characterized by their higher complexity in terms of location and distribution of DHSRs; the other is situated approximately 600 bp downstream of the MCP-1 specific polyadenylation signal within a 3'-regulatory domain, whose chromatin structure only became accessible after cytokine treatment.

### EXAMPLE 5: DNA sequencing and band-shift analysis led to the detection of a novel AP-1 site within the 3'-DNAse I hypersensitive region

Since the MCP-1 gene is located at chromosome 17 a chromosome-specific library was screened to isolate the whole genomic locus of the human MCP-1 gene using the corresponding cDNA (Rollins et al., 1989, Mol. Cell. Biol. 9, p. 4687-4695) and a PCR amplified promoter fragment (Rösl et al., 1994, J. Virol. 68, p. 2142-2150) as hybridization probe. DNA sequence analysis essentially confirmed the sequence data of the 3.8 kb upstream region published by Ueda and co-workers (Ueda et al., 1994) (see also Fig. 6). Single nucleotide variations may be dependent on different origins of the clones used for scquencing. Inspecting the nucleotide sequence surrounding the minor 5'-DHSR 1.5 kb upstream of the Kpn I site, a putative NF-IL-6 (C/EBP-β) consensus sequence was found (Fig. 6). Similar sequences could be detected within the minor 2.0 kb subfragment and the major 3'-end DHSR 2.7-3.4 bp subfragments (see Fig. 6). The nucleotide stretch covered by the major DHSR in native "444"-chromatin, where two contiguous NF-κB sites and one AP-1 site are present, could be detected (Fig. 6). Since all three cis-regulatory sequences were shown to be functional in DNA binding assays, they were not further followed up in this experiment. Both the distal 5.1 kb and the proximal 1.5 kb minor 5'-DHSRs coincide with putative consensus sequences for the transcription factor Sp 1.

Scanning the 3'-end for potential regulatory sequences, an additional downstream AP-1 site (5'-TGAGTCA-3'; pos. +2594/+2600) (Fig. 6) could be detected. To prove the functionality of this sequence in terms of DNA binding, electrophoretic band-shift assays were performed. Using the oligonucleotide 5'-GGAAGGTTGAGTCAAGGGATT-3' (pos. +2587/+2607), a single band can be visualized after incubation with a nuclear extract obtained from "444"-cells (Fig. 7A, lane 1). The specificity of AP-1 binding was confirmed in competition experiments by adding a 100-fold molar unlabeled excess of either the homologous oligonucleotide (lane 2; "hom. comp. AP-1 3'", 5'-GGAAGGTTGAGTCAAGGGATT-3') or a oligonucleotide containing the AP-1 site (lane 4; "comp. AP-1 consensus", 5'-CGCTTGATGACTCAGCCGGAA-3') of the collagenase TPA responsive element, leading in both cases to complete disappearance of the 3'-AP-1 retarded band. No competition, however, could be achieved, when a heterologous, nuclear factor 1 ("NF-1") specific oligonucleotide (lane 3; "hetero.comp. NF-1", 5'-TTTTGGATTGAAGCCAATATGATAA-3') was applied. In order to discern whether or nor the extent of AP-1 binding is different between "444"- and "CGL3"-cells and whether the affinity was modulated by TNF-α, the binding properties of different extracts were analysed in the same polyacrylamide gel (Fig. 7B). Although "CGL3"-cells lack MCP-1 expression (see Fig. 1 and 2), all nuclear extracts showed identical binding affinity independently of whether the cells were treated with TNF-α or not prior to cell harvesting (Fig. 7B, lanes 1-4). The absence of enhanced AP-1 binding was not an exceptional feature of the 3'-AP-1 site, because the affinity to the collagenase specific site was also significantly altered (Fig. 7B, lanes 5 and 6).

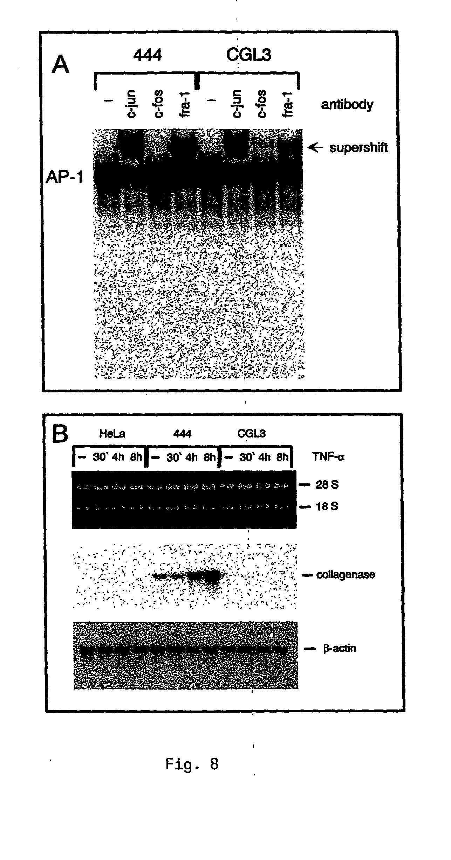

Since it could previously be demonstrated that the composition of AP-1 differs considerably with respect to the *jun*/*fos* and the *jun*/*fra-1* ratio in tumorigenic and non-tumorigenic cells, band-shift assays in combination with specific antibodies raised against AP- 1 family members were carried out. Antibodies which have been used recognized c-Fos (epitope corresponding to amino acids 3-16 mapping at the N-terminus of human c-Fos protein), Fra-1 (epitope corresponding to amino acids 3-22 mapping at the N-terminus of the human Fra-1 protein), c-Jun (epitope corresponding to amino acids 56-69 mapping within the carboxy-terminal domain of the mouse c-Jun protein; recognizes both the unphosphorylated and phosphorylated form of c-Jun). All antibodies were obtained from Santa Cruz Biotechnology (Santa Cruz, California, USA) as "TransCruz" supershift reagents (c.f. Soto et al. (1999), Oncogene 18, p. 3187-3198.) As illustrated in Fig. 8A, addition of a *c-jun* antibody resulted in a strong super-shift indicating that there exists substantial amounts of *c-jun* homo- and heterodimers in both cell lines (lanes 2 and 6). A completely different situation, however, was obtained when antibodies against *fra-1* were added after the binding reaction. In "444"-cells, most (50%) of *jun*-family members (e. g. c-jun) were mainly heterodimerized with the fos-related protein *fra-1* (Fig. 9A, lane 4), while in tumorigenic "CGL3"-cells only approximately 15% of the radioactivity (as determined by quantification of the radioactivity in a "Phospholmager") was retarded within the super-shift complex (lane 8). Conversely, AP-1 in "CGL3"-cells contain *c-fos* (between 5-7%) (lane 7), which is not detectable in non-malignant "444"-cells (lane 3), even after prolonged exposure to the autoradiography. EMSA supershift pattern was not significantly altered after TNF-α addition. To unequivocally demonstrate a different functionality of the transcription factor AP-1 upon cytokine addition in the experimental cell system, the expression of a marker gene which is known to be tightly regulated by AP-1 was studied. As already shown for MCP-1 (Fig. 1, panel A), treatment with TNF-α resulted in a strong induction of the type I collagenase exclusively in "444"-cells, while the gene was transcriptionally silent in the tumorigenic segregants of the same hybrids ("CGL3") or in the parentat HeLa cells (Fig. 8, panel B).

## Claims

1. A nucleic acid molecule comprising
(a) a nucleic acid sequence encoding the monocyte-chemoattractant-protein-1 (MCP-1) encoded by the EMBL clone Y 18933 or a protein having at least 80 % amino sequence identity to said protein; and
(b) a 3'-DNAse I-hypersensitive region (DHSR) comprising a nucleic acid molecule which is located 2430 bp to 3019 bp downstream of the transcriptional start site of the MCP-1 gene and has at least 80% identity with the nucleic acid sequence of SEQ. ID.NO:1, wherein said 3'-DNAse I-hypersensitive region comprises the nucleic acid sequence TGAGTCA.

2. The nucleic acid molecule of claim 1, wherein the 3'-DNAse I-hypersensitive region comprises the nucleic acid sequence of SEQ ID NO:1.

3. The nucleic acid molecule of claim 1, wherein the 3'-DNAse I-hypersensitive region comprises the nucleic acid sequence GGAAGGTTGAGTCAAGGATT.

4. The nucleic acid molecule of any one of the claims 1 to 3 wherein the hypersensitivity sequence (b) contains mutations resulting in a modified DNAse I hypersensitivity, S1 hypersensitivity and/or altered interaction with transcription factors, wherein the identity of the mutated sequence with the nucleic acid sequence of the 3'-DNAse I-hypersensitive region is at least 80% with the nucleic acid sequence of SEQ ID NO:1.

5. The nucleic acid molecule of claim 4, wherein the transcription factor is AP-1 or NF-IL6.

6. A recombinant vector containing the nucleic acid molecule of any one of claim 1 to 5.

7. The recombinant vector of claim 6 wherein the nucleic acid molecule is operatively linked to regulatory elements allowing transcription and synthesis of a translatable RNA in prokaryotic and/or eukaryotic host cells.

8. A recombinant host cell which contains a nucleic acid molecule according to any one of claims 1 to 5 or the recombinant vector of claim 6 or 7.

9. The recombinant host cell of claim 8, which is a mammalian cell, a bacterial cell, an insect cell or a yeast cell.

## Patentansprüche

1. Nukleinsäuremolekül umfassend
(a) eine Nukleinsäuresequenz, die das vom EMBL Klon Y 18933 kodierte Monozyten-Chemotaktische-Protein-1 (MCP-1) oder ein Protein mit einer zumindest 80 % identischen Aminosäuresequenz zu diesem Protein kodiert; und
(b) eine 3'-DNAse I-hypersensitive Region (DHSR), die ein Nukleinsäuremolekül umfasst, das von 2430 bp bis 3019 bp abwärts von der Transkriptionsstartstelle des MCP-1 Gens lokalisiert ist und zumindest 80% identisch mit der Nukleinsäuresequenz von SEQ ID NO:1 ist, wobei die 3'-DNAse I-hypersensitive Region die Nukleinsäuresequenz TGAGTCA umfasst.

2. Nukleinsäuremolekül nach Anspruch 1, wobei die 3'-DNAse I-hypersensitive Region die Nukleinsäuresequenz der SEQ ID NO:1 umfasst.

3. Nukleinsäuremolekül nach Anspruch 1, wobei die 3'-DNAse I-hypersensitive Region die Nukleinsäuresequenz GGAAGGTTGAGTCAAGGATT umfasst.

4. Nukleinsäuremolekül nach einem der Ansprüche 1 bis 3, wobei die hypersensitive Sequenz (b) Mutationen enthält, die zu DNAse I Hypersensitivität, S1 Hypersensitivität und/oder veränderter Interaktion mit Transkriptionsfaktoren führen, wobei die mutierte Sequenz zumindest 80% identisch mit der Nukleinsäuresequenz der 3'-DNAse I-hypersensitiven Region mit der Nukleinsäuresequenz SEQ ID NO:1 ist.

5. Nukleinsäuremolekül nach Anspruch 4, wobei der Transkriptionsfaktor AP-1 oder NF-IL6 ist.

6. Rekombinanter Vektor enthaltend das Nukleinsäuremolekül nach einem der Ansprüche 1 bis 5.

7. Rekombinanter Vektor nach Anspruch 6, wobei das Nukleinsäuremolekül operativ verbunden ist mit regulatorischen Elementen, die Transkription und Synthese einer translatierbaren RNA in prokaryotischen und/oder eukaryotischen Wirtszellen erlauben.

8. Rekombinante Wirtszelle, die ein Nukleinsäuremolekül nach einem der Ansprüche 1 bis 5 oder den rekombinanten Vektor nach Anspruch 6 oder 7 enthält.

9. Rekombinante Wirtszelle nach Anspruch 8, die eine Säugerzelle, eine bakterielle Zelle, eine Insektenzelle oder eine Hefezelle ist.

## Revendications

1. Molécule d'acide nucléique comprenant :
- une séquence d'acide nucléique encodant la protéine-1 monocyte chimioattirant (MCP-1) encodée par le clone EMBL Y 18933 ou bien une protéine ayant au moins 80% de séquences amino identiques à ladite protéine, et
- une région hypersensible-I de 3'-ADNse (DHSR) comprenant une molécule d'acide nucléique qui est disposée de 2430 bp à 3019 bp en aval du site de départ transcriptionnel du gène MCP-1 et possède au moins 80% d'identité avec la séquence d'acide nucléique de SEQ. ID.NO :1, dans laquelle ladite région hypersensible-I de 3'-ADNse comprend la séquence de l'acide nucléique TGAGTCA.

2. Molécule d'acide nucléique selon la revendication 1, **caractérisée en ce que** la région hypersensible-I de la 3'-ADNse comprend la séquence d'acide nucléique de SEQ ID NO :1.

3. Molécule d'acide nucléique selon la revendication 1, **caractérisée en ce que** la région hypersensible-I de 3'-ADNse comprend la séquence d'acide nucléique GGAAGGTTGAGTCAAGGATT.

4. Molécule d'acide nucléique selon l'une quelconque des revendications 1 à 3, **caractérisée en ce que** la séquence d'hypersensibilité (b) incorpore des mutations résultant de l'hypersensibilité-I de DNAse modifiée, de l'hypersensibilité S1 et/ou une interaction altérée avec des facteurs de transcription, dans laquelle l'identité de la séquence mutée avec la séquence d'acide nucléique de la région hypersensible-I de 3'-ANDse est au moins de 80% avec la séquence d'acide nucléique de SEQ ID NO :1.

5. Molécule d'acide nucléique selon la revendication 4, **caractérisée en ce que** le facteur de transcription est AP-1 ou NF-IL6.

6. Vecteur recombinant renfermant une molécule d'acide nucléique selon l'une quelconque des revendications 1 à 5.

7. Vecteur recombinant selon la revendication 6, **caractérisé en ce que** la molécule d'acide nucléique est liée opérativement à des éléments régulateurs permettant la transcription et la synthèse d'un ARN translatable dans des cellules hôtes procariotiques et/ou eucariotiques.

8. Cellule hôte recombinante renfermant une molécule d'acide nucléique selon l'une quelconque des revendications 1 à 5 ou le vecteur recombinant selon la revendication 6 ou 7.

9. Cellule hôte recombinante selon la revendication 8, qui est une cellule mammifère, une cellule bactérienne, une cellule d'insecte ou une cellule de levure.
